# EUROPEAN PATENT SPECIFICATION

(11) **EP 3 100 707 B1**
(45) Date of publication and mention of the grant of the patent: **27.12.2017**
(21) Application number: 16172203.8
(22) Date of filing: 13.11.2012
(51) Int. Cl.: A61H 1/02, A61H 39/04

(54) **APPARATUS FOR LOCOMOTION THERAPY**
VORRICHTUNG ZUR BEWEGUNGSTHERAPIE
APPAREIL DE THÉRAPIE DE LOCOMOTION

(43) Date of publication of application: 07.12.2016
(62) Divisional of application: 12192459.1
(73) Proprietor: Hocoma AG, 8604 Volketswil (CH)
(72) Inventor: BUCHER, Rainer, 8907 Wettswil (CH); DAREMAS, Konstantin, 8352 Elsau (CH)
(74) Representative: Sticht, Andreas

(56) References cited:
- EP-A2- 0 205 018
- EP-B1- 1 169 003
- US-A- 2 695 017
- US-A- 4 535 762
- US-A- 4 662 681
- US-A- 5 267 924
- US-B1- 7 097 628

## Description

The present invention relates to an apparatus for locomotion therapy which enables to start locomotion training in an early stage of rehabilitation therapy.

In incompletely paraplegic patients the possibility has been shown to exist of improving walking ability up to normality by means of adequate locomotion training. To this end, an apparatus has been developed to provide an intensive walking training (activation of the central pattern generator in the spinal cord) of paraparetic and hemiparetic patients in early stages of rehabilitation when they are for example in a still unstable circulatory situation. Such an apparatus is for example described in EP 1 169 003 B1. In the apparatus described in this document, a table is provided which may be moved gradually between a horizontal position corresponding to a horizontal position of a patient to a vertical position corresponding to an upright position of the patient. Furthermore, in this apparatus a knee mechanism and a foot mechanism are provided, the knee mechanism being able to move the knees and therefore the legs of a person without the person himself having to exert any effort.

In this apparatus, the foot mechanism as well as the knee mechanism have an adjustable position on the table to be able to adjust the apparatus to various heights of patients. However, depending on the height of the patient such an adjustment may result in the patient being relatively high above the ground in a vertical position of the table, which for example may make it more difficult for a therapist to care for the person, which is in particular needed if the person is in an unstable circulatory condition or otherwise still seriously impaired.

Therefore, it is an object of the present invention to provide an apparatus for locomotion therapy of the general kind described in EP 1 169 003 B1 in which a position of a person in the vertical position of the apparatus may be kept low.

In this respect, an apparatus as defined in claim 1 is provided. The dependent claims define further embodiments, at least some of which define further improvements of the above-mentioned apparatus of EP 1 169 003 B1.

According to an embodiment, an apparatus for locomotion therapy is provided, further comprising:
a second table portion coupled to said first table portion, a position of said second table portion being adjustable with respect to said first table portion to adapt the apparatus to different heights of patients.

In such an apparatus, using the tilting mechanism a table formed by the first table portion and the second table portion may be moved between a horizontal position and a vertical position. As the foot mechanism is mounted to the first table portion, and the adjustment to a height of the patient is performed by adjusting the position of the second table portion which is coupled to the first table portion, the position of the foot mechanism in particular in a vertical position is independent of an adjustment of the apparatus to the size of the person, which may keep a position of the patient low and make it easier for therapists to care for the patients. In other words, the second table portion in such embodiments is connected to the tilting mechanism only via the first table portion, but not directly.

In an embodiment, the tilting mechanism may tilt the first table portion about an axis coupling the first table portion with the tilting mechanism. Furthermore, in an embodiment, the apparatus additionally comprises a lifting mechanism to move said first table portion, possibly together with said tilting mechanism, in a vertical direction.

In addition to the adjustment of the second table portion with respect to the first table portion for height adjustment, the second table portion may be tiltable with respect to the first table portion to selectively adjust a hip extension or hip flexion.

In some embodiments, the foot mechanism may comprise a first footrest and a second footrest. In an embodiment, the first and second footrests may comprise load sensors to measure a load the patient exerts on the footrest. Alternatively or additionally, other load sensing mechanisms may be provided to measure the load. In other embodiments, additionally or alternatively to the load sensors the first and second footrests may comprise mechanical stimulating elements for selective mechanical stimulation of areas of the feet of the patient. This mechanical stimulation may be performed synchronously with a movement of the leg movement mechanism.

The foot mechanism may further comprise a first base and a second base, the first and second bases being mounted to the first table portion, for example slidably mounted to the first table portion to accommodate movements of the legs. The first footrest may be mounted to the first base portion via a first double joint, and the second footrest may be mounted to the second base portion via a second double joint. Via such double joints, which may comprise a rod, a plate or other elongated element between the two joints, one end of the rod being coupled with the first footrest at a tip portion thereof via a first joint of the double joint and a second end of the rod being coupled with the base portion via a second joint of the double joint, a natural movement of the foot may be improved. In some embodiments, the natural movement may be additionally improved by generating an additional stimulus on the foot through an impact when the footrest is decelerated or stopped at a stopper. This stopping may be performed using a resilient stopper like a spring to adjust the force acting on the foot.

It should be noted that the above-described foot mechanisms may also be used in other apparatuses for locomotion therapy than the one described above.

The apparatus may further comprise an electrical stimulation device which may be configured to electrically stimulate muscles, in particular leg muscles, of the person synchronously with a movement of the leg movement mechanism.

The leg movement mechanism comprises a first mechanism for a left leg and a second mechanism for a right leg of the patient. A distance between the first mechanism and the second mechanism is adjustable to accommodate the leg movement mechanism to a "track width" of the patient. Such an adjustment may also be used independently of the apparatus described above, i.e. may also be used in other apparatuses for locomotion therapy.

In some embodiments, a pelvic harness may be fixed to said first table portion and/or said second table portion, in particular said second table portion. Additionally, shoulder straps may be provided to fix a torso of the patient to the apparatus.

The adjustment of the leg movement mechanism may be performed while the first and second table portions are in a horizontal position, i.e. while the patient is lying on the table. By fixing the pelvic region of the patient via the pelvic harness and adjusting the leg mechanism accordingly, a correct centre of rotation may be provided for the leg movement, thus avoiding or reducing shearing forces which may occur when the mechanical centre of rotation and the orthopedically correct center of rotation deviate from each other.

Further features and embodiments will become apparent from the following detailed description with reference to the attached drawings, wherein:
Fig. 1 is a side view of an apparatus according to an embodiment;
Fig. 2 is a perspective view of the apparatus of Fig. 1;
Fig. 3 is a perspective view of the apparatus of Fig. 1 with a table in an intermediate position;
Fig. 4 is a side view of the apparatus of Fig. 1 with the table in a near vertical position;
Fig. 5 is a side view of the apparatus of Fig. 1 with the table in a vertical position;
Fig. 6 is a partial view of an apparatus according to an embodiment for illustrating an adjustment of a leg movement mechanism;
Fig. 7 is a partial view of an apparatus according to an embodiment illustrating an embodiment of a foot mechanism;
Fig. 8 is a diagram illustrating loading of an apparatus according to an embodiment;
Fig. 9A-9C show examples for a foot stimulation;
Fig. 10 is a schematic diagram showing various muscles which may be stimulated in an embodiment;
Fig. 11 shows a block diagram of some features of some embodiments;
Fig. 12A-12C show a particular embodiment of a foot mechanism in three different positions;
Fig. 13A and 13B show pelvic harnesses usable in some embodiments;
Fig. 14 is a side view of an apparatus according to an embodiment illustration an emergency lowering mechanism;
Fig. 15 is a perspective view of an apparatus according to an embodiment; and
Fig. 16 is a further perspective view of the apparatus of Fig. 15.

In the following, embodiments of the present invention will be described in detail with reference to the attached drawings. It should be noted that these embodiments are given only for the purpose of illustration and are not to be construed as limiting the scope of this application.

It should be noted that features of different embodiments may be combined with each other unless specifically noted otherwise. Furthermore, describing an embodiment with a plurality of features is not to be construed as indicating that all those features are necessary for practicing the present invention, as other embodiments may comprise less features and/or alternative features to the ones shown in the drawings or described herein.

Various components of the embodiments shown are not necessarily drawn to scale with each other, but are depicted in a manner to provide a clear understanding of the respective embodiment.

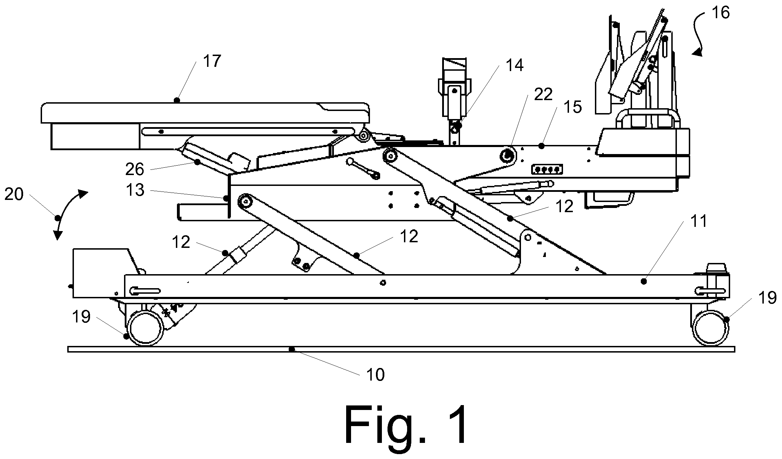

Turning now to the figures, in Fig. 1-5 various views of an apparatus for locomotion therapy according to an embodiment are shown. In particular, Fig. 1 shows a side view of the apparatus with a table comprising a first table portion 15 and a second table portion 17 being in a horizontal position. In other Figures, other positions of the apparatus are shown. It should be noted that training is possible in various positions, e.g. in the horizontal position shown in Fig. 1 or in tilted or vertical positions described later.

The apparatus of Fig. 1 further comprises a base frame, i.e. a base 11 having wheels 19 for rolling the apparatus on a floor 10. However, in other embodiments, the apparatus may be stationary without having wheels. First table portion 15 is coupled with base frame 11 via a lifting mechanism 12 and a tilting mechanism 13. Using lifting mechanism 12 which comprises rods and actuators, tilting mechanism 13 together with first and second table portions 15, 17 may be lifted in a vertical direction, in this case following an arc as indicated by an arrow 20, i.e. also with a movement component in the horizontal direction.

Furthermore, as will be explained further below, for tilting the table formed by first table portion 15 and second table portion 17 between the horizontal position shown in Fig. 1 and a vertical position, first table portion 15 may be rotated about an axis 22 by tilting mechanism 13. It should be noted that the shown lifting and tilting mechanisms 12, 13 are merely an example, and in other embodiments other mechanisms, for example using more than one axis, may be used to lift and/or tilt first table portion 15 with respect to base 11.

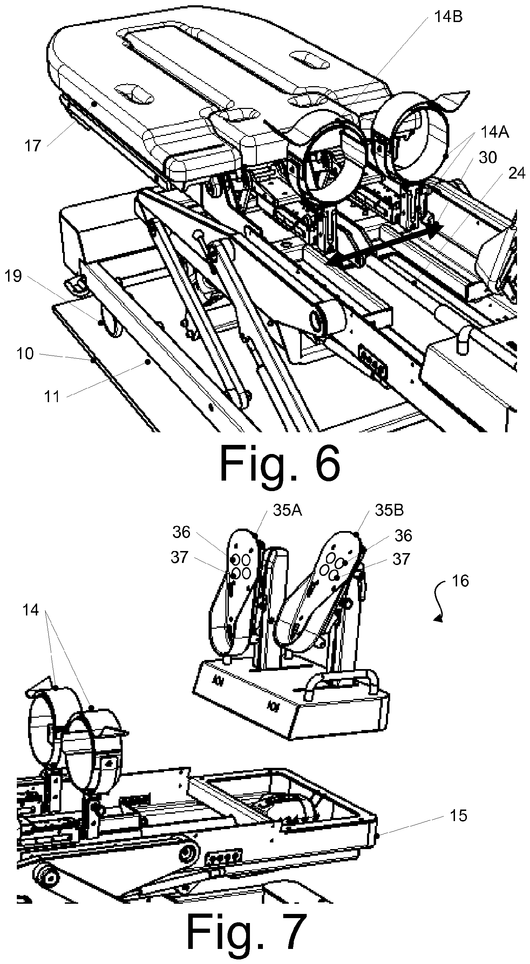

Furthermore, a foot mechanism is mounted to first table portion 15 and configured to receive feet of a patient using the apparatus. Also, a leg movement mechanism 14 which may comprise belts as shown to fix the legs of the person to the mechanism is provided for moving legs of a person using the apparatus, in particular to flex knees of the person to emulate a walking movement, to give an example. The belts may be adjustable to accommodate leg movement mechanism 14 to different leg diameters. Suitable leg movement mechanisms are described in the above-mentioned EP 1 169 003 B1 in detail and will not be described in greater detail here except for an additional adjustment possibility described later with respect to Fig. 6.

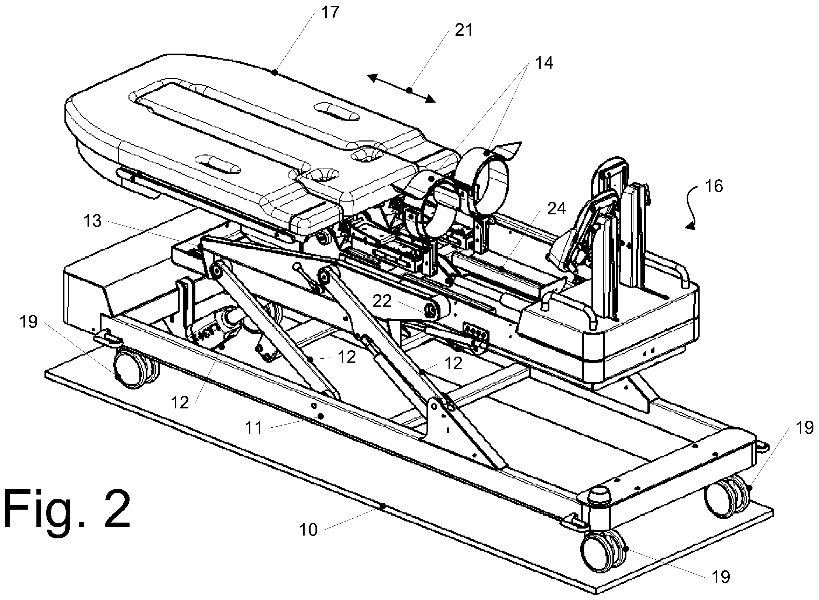

Fig. 2 shows a perspective view of the apparatus of Fig. 1 in the same position, i.e. tilting mechanism 13 being slightly lifted via lifting mechanism 12 and first and second table portions 15, 17 being in a vertical position. As can be seen in Fig. 2, second table portion 17 is coupled to first table portion 15 via a drive mechanism 24, for example a linear drive. Using drive mechanism 24, the position of second table portion 17 may be adjusted as indicated by an arrow 21 with respect to first table portion 15 to adapt the apparatus to patients of different heights. For example, for taller patients, second table portion 17 which in operation for example accommodates back and head of a person may be moved away from first table portion 15, and for smaller persons it may be moved towards first table portion 15. As first table portion 15 is mounted to tilting mechanism 13 to tilt about axis 22, such an adjustment to accommodate different heights of patients does not change the distance between foot mechanism 16 and axis 22 and therefore, as will be explained in more detail later, helps to keep the position of foot mechanism 16 and therefore of the patient low when tilting first table portion 15.

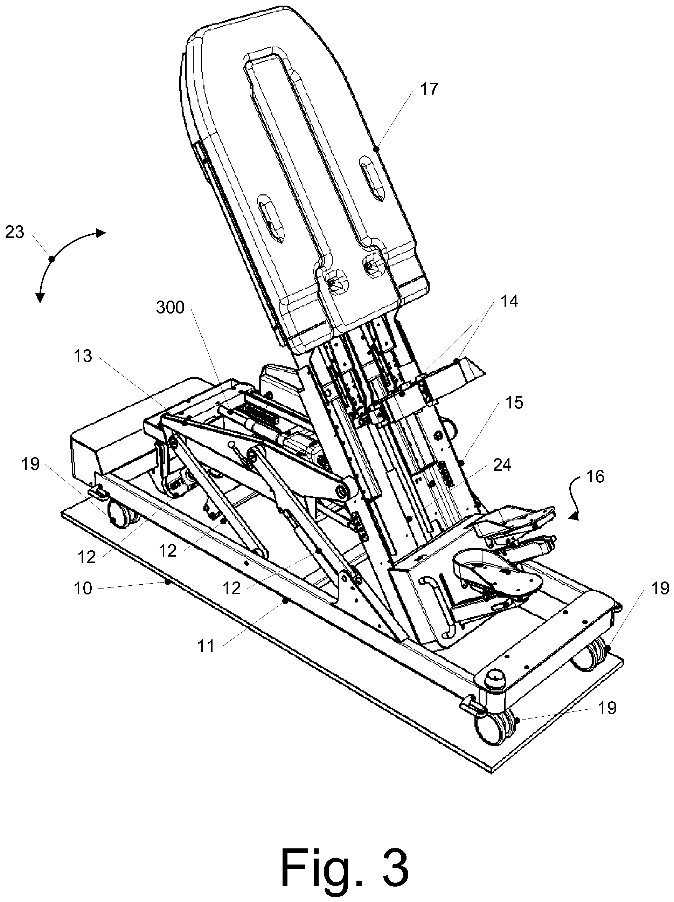

The result of such a tilting motion is shown in Fig. 3. Here, lower table portion 15 has been tilted about axis 22 as indicated by an arrow 23 by tilting mechanism 13, in particular by using a drive 300 of tilting mechanism 13, to a position between a horizontal position and a vertical position, for example a position of about 45 degrees.

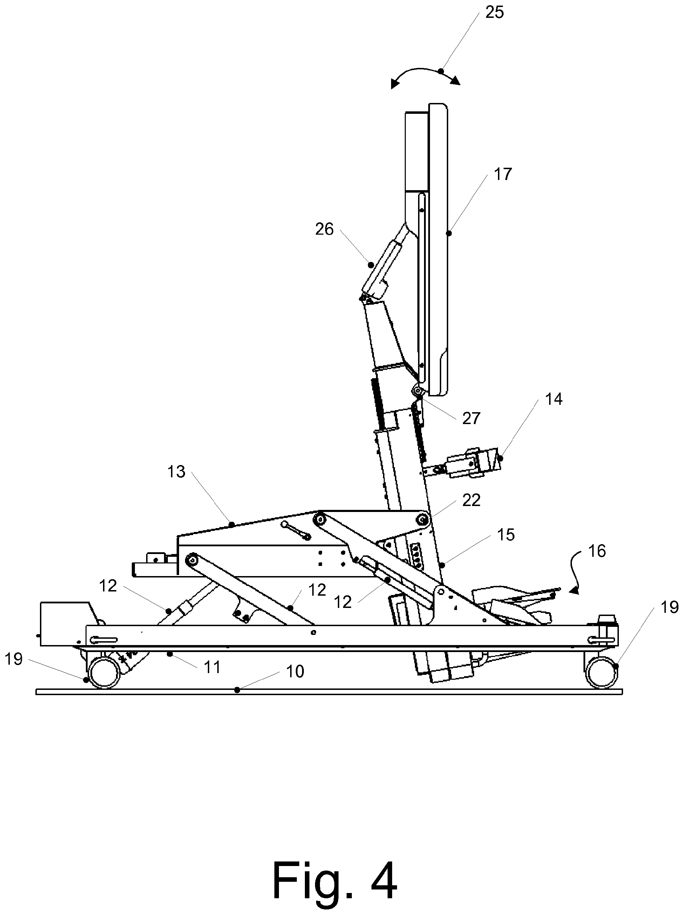

In Fig. 4, the first table portion 15 and therefore also the second table portion 17 connected thereto are shown in an almost vertical position. Also, as shown in Fig. 4 using a mechanism 26, for example a further linear drive, second table portion 17 may be tilted as indicated by an arrow 25 with respect to first table portion 15. In this way, an extension or flexion of a hip joint of a patient may be adjusted (hereinafter shortly referred to as "hip extension" and "hip flexion", respectively). A hip extension may for example be increased by tilting second table portion 17 backwards, i.e. to the left side in Fig. 4, while a hip flexion may be increased by tilting second table portion 17 forward, i.e. to the right side in Fig. 4. In some embodiments, in this way even an "overhanging" patient position may be reached if desired. In other embodiments, mechanism 26 may be omitted, for example be replaced by a rigid element, such that the angular relationship between first and second table portions 15, 17 is fixed.

Also, as can be seen in Fig. 4, foot mechanism 16 is close to floor 10, which is achieved by tilting first table portion 15 and performing any height adjustment by adjusting the position of second table portion 17 relative to first table portion 15. Adjusting the vertical position of tilting mechanism 13 and therefore of axis 22 may additionally contribute to keep foot mechanism 16 and therefore the patient close to floor 10.

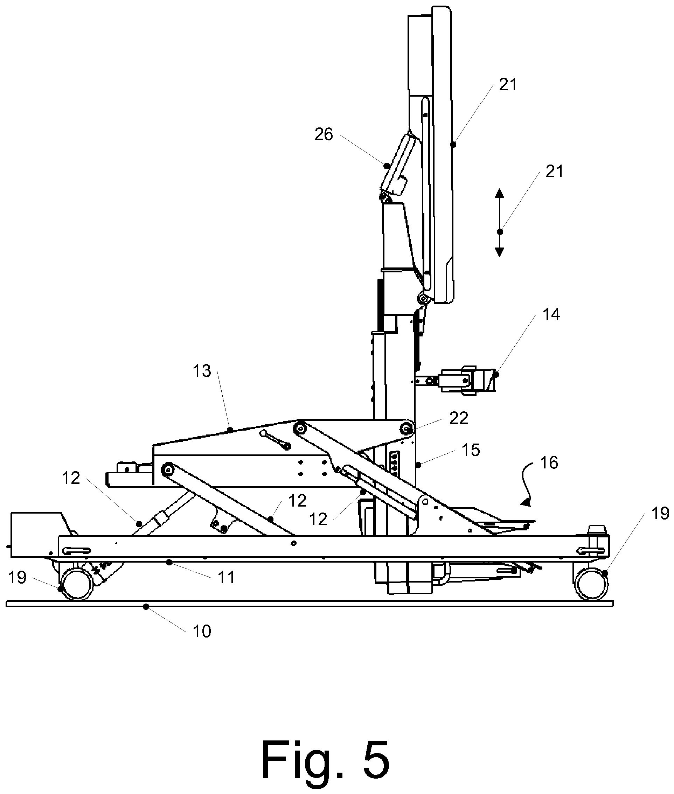

Finally, in Fig. 5 first table portion 15 is tilted to a completely vertical position. It should be noted that also in this position or in all intermediate positions, second table portion 17 may be adjusted with respect to first table portion 15 as indicated by arrow 21.

It should be noted that the various drive mechanisms used for performing the adjustments and movements described with reference to Fig. 1-5 may comprise electric motors, pneumatic elements or other actors. It is also conceivable that some of these elements are adjusted manually, for example by providing a crank lever and a corresponding gear mechanism.

In the following, with respect to Fig. 6-16, various optional additional features which may in some embodiments be implemented in the apparatus of Fig. 1-5 will be described. It should be noted that these optional additional features may not only be implemented in the apparatus of Fig. 1-5, but also in other apparatuses for locomotion therapy which enable a movement or tilting of a person between a horizontal position and a vertical position, for example the apparatus of the above-mentioned EP 1 169 003 B1. It should further be noted that the various features described with reference to Fig. 6-12 may be used independently from each other, but also combinations of some or all of these features may be used. Nevertheless, for ease of reference these optional features will be described with respect to the embodiments of Fig. 1-5.

In Fig. 6, an optional adjustment possibility for a leg movement mechanism is shown. In the embodiment of Fig. 6, the leg movement mechanism comprises a first mechanism 14Afor a left leg of the patient and a second mechanism 14B for a right leg of the person. These mechanisms themselves may work as in the above-mentioned EP 1 169 003 B1, e.g. move to flex and extend a knee of a person and for example be driven by eccentric drive. As indicated by an arrow 30 in Fig. 6, a distance between first and second mechanisms 14A and 14B is adjustable to accommodate the apparatus to different "track widths" of patients. Such an adjustment may for example be provided by mounting leg mechanisms 14A, 14B slidably in tracks or bearings or using a drive mechanism using a gear rack. However, other adjustment mechanisms are also possible.

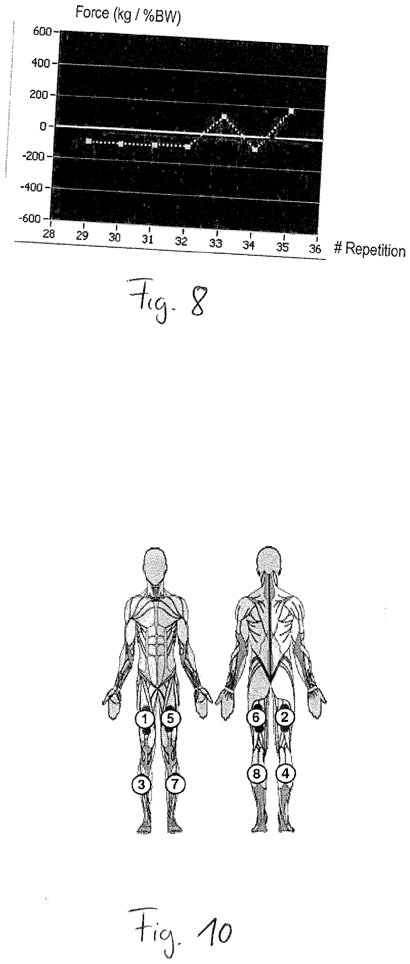

In Fig. 7, a specific implementation of a foot mechanism 16 is shown. In the embodiment of Fig. 7, foot mechanism 16 comprises a first footrest 35A for a left foot of a person and a second footrest 35B for a right foot of the person. In footrests 35A, 35B load sensors 36 may be provided to measure a load caused by the patient, i.e. a force which is exerted by the patient on footrest 35A and/or 35B. With cyclic exercises like a cyclic flexion and extension of the knee the force relative to the body weight may be plotted over the number of repetitions as shown as an example in Fig. 8. In such a way, a good control and documentation of the therapy is possible.

In other embodiments, instead of or in addition to load sensors 36 provided in footrests 35A, 35B other kind of load sensing mechanisms may be used for measuring the load. For example, a load may be measured indirectly by measuring a displacement of footrests 35A, 35B caused by a load e.g. by measuring an angle or using potentiometers and taking a restoring force of springs or other resilient elements biasing footrests 35A, 35B towards a predetermined standby position into account, or by sensing a force or a torque at another point in the foot mechanisms than directly at footrests 35A, 35B.

In addition to sensors 36 or as an alternative thereto, footrests 35A, 35B may also comprise mechanical stimulation elements 37. Through such mechanical stimulation elements, pressure may be exerted locally and selectively to various areas of the soles of the feet of the person. Such a stimulation may in particular be performed synchronously with the movement of the leg movement mechanism, such that the mechanical stimulation of the soles of the feet may simulate the feeling of rolling the foot from heel to toe while walking, thus being in synchronicity with the walking movement emulated by the leg movement mechanism. It is to be noted that such a synchronous control of the leg movement mechanism and the mechanical stimulation of the soles of the feet is not restricted to cyclic movements or stimulations, but also other kinds of coordinated control also are considered as being a synchronous control within the scope of this application. For example, foot stimulations simulating a stumbling may be inserted at certain points of the movement of the leg movement mechanism, or the leg movement mechanism itself may be controlled to simulate a stumbling or other event.

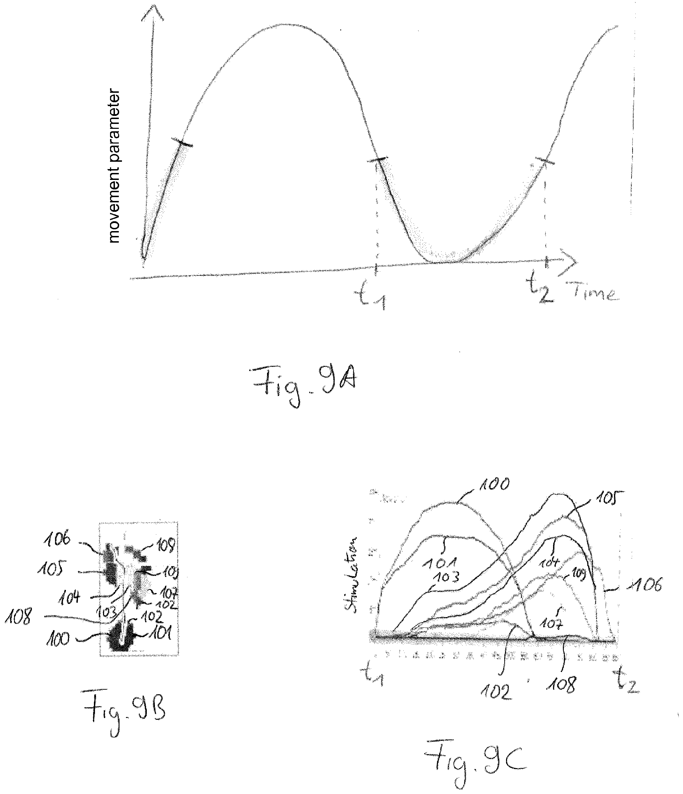

In particular, possibilities for foot stimulations will now be explained with reference to Fig. 9 in more detail. Fig. 9A shows a movement parameter like an amplitude or a position of a leg movement mechanism over time, a high amplitude for example corresponding to a flexed knee and a low amplitude corresponding to a more extended knee. Afoot stimulation may for example be performed in portions with lower amplitude, for example between times t₁ and t₂ of each cycle of the leg movement mechanism. The remaining time, for example the higher amplitudes, may for example correspond to phases of the walking movement where the foot would be lifted from the ground. Consequently, in these times no foot stimulation is performed, i.e. no additional pressure is exerted on the foot.

Between t₁ and t₂, stimulation may start at the heel and then move to the tip of the foot. Heel and tip of the foot may be divided in different portions which are stimulated at different times and/or with different intensities. An example for this is shown in Fig. 9B and 9C, where Fig. 9B shows various zones of a foot (in this case a right foot) and Fig. 9C shows corresponding stimulation curves for the zones. A zone and its associated stimulation curve (intensity of stimulation over time) bear the same reference numeral 100-109. However, the partitioning of the foot into zones and the stimulation curves shown in Fig. 9C serve only as examples, and in other embodiments, other partitionings or stimulation curves, for example partitionings with less zones or more zones, are possible.

In some embodiments, also an electrical stimulation of muscles of the patient, in particular leg muscles, is used. Such an electrical stimulation is also known as functional electrical stimulation (FES).

For example, in Fig. 10, a front view and a back view of a person are schematically shown with muscles groups marked by 1-8. These muscle groups may for example be stimulated synchronously with the leg movement mechanism 14 of the preceding embodiments.

For example, regarding the muscles groups shown in Fig. 10, during a phase of increasing amplitude of the leg movement as shown in Fig. 9A (positive slope), muscles groups 2, 4, 6 and 8 may be stimulated, and during a phase of falling amplitude (negative slope) of Fig. 9A muscles groups 1, 3, 5 and 7 may be stimulated via electrodes to which a corresponding voltage is applied. However, this stimulation scheme serves only as an example, and for example in other embodiments only some of the muscle groups depicted may be stimulated.

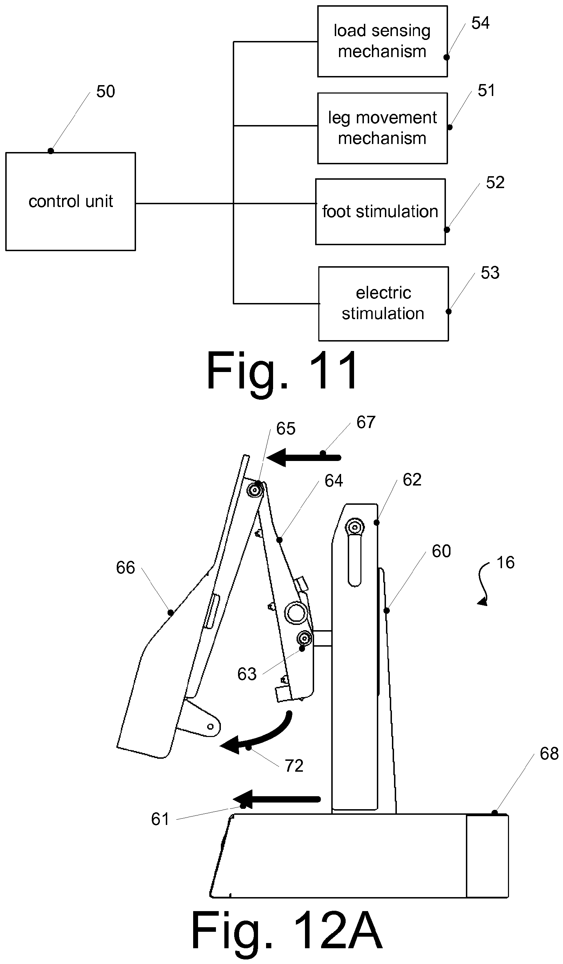

In Fig. 11, a block diagram of a part of an embodiment employing foot stimulation and electric stimulation is depicted. A control unit 50, for example a correspondingly programmed microprocessor, controls a leg movement mechanism 51, for example leg movement mechanism 14 of the embodiments of Fig. 1-5. Additionally, control unit 50 controls a foot stimulation 52, for example the foot stimulation explained with reference to Fig. 7 and 9, synchronously with leg movement mechanism 51. Additionally or alternatively to foot stimulation 52, control unit 50 may also control an electric stimulation 53 synchronously with leg movement mechanism 51, for example as explained with reference to Fig. 10. It should be noted that in other embodiments, foot stimulation 52 and electric stimulation 53 may be omitted. Additionally, control unit 50 may receive signals from a load sensing mechanism 54 which may be implemented as described above with reference to Fig. 7.

Next, with reference to Fig. 12A-12C a specific embodiment of a foot mechanism 16 will be described. This foot mechanism may be employed in the embodiment of Fig. 1-5, but may also be employed in other apparatuses for locomotion therapy. Moreover, the foot mechanism of Fig. 12A-12C may be combined with the load sensors and/or the mechanical foot stimulation of Fig. 7, but may also be used without these features.

Fig. 12A-12C show foot mechanism 16 in three different positions corresponding to three different phases of a cycle of the leg movement mechanism, for example. While in Fig. 12A-12C only a single mechanism for one foot is shown, two such mechanisms may be provided, one for the left foot and one for the right foot of the patient.

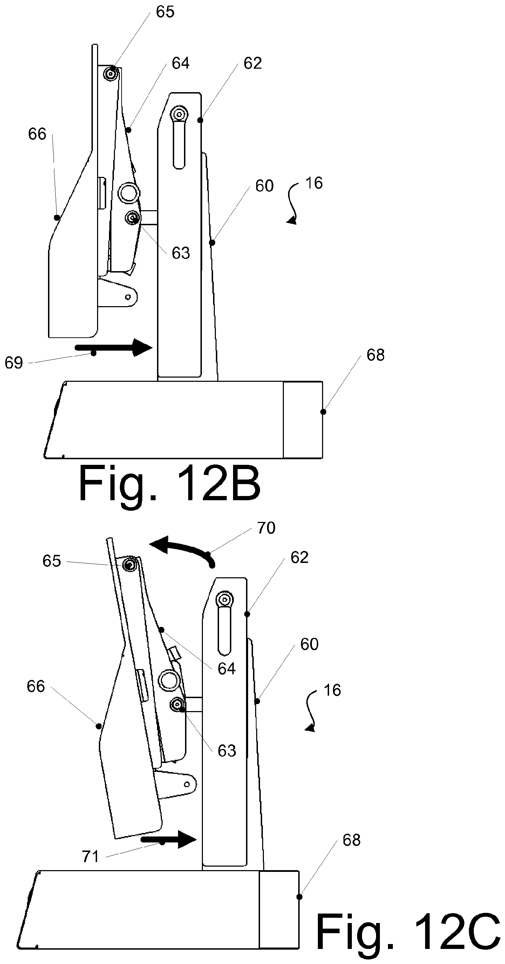

The foot mechanism 16 of Fig. 12A-12C comprises a mounting portion 68 to be mounted for example to first table portion 15 of the embodiment of Fig. 1-5. For the mechanism, a base portion 60 in form of a ridge is slidably mounted on mounting portion 68. Fixed to base portion 60 is an intermediate portion 62. A footrest 66 is coupled with intermediate portion 62 and therefore with base portion 60 via a double joint comprising, in the embodiment of Fig. 12A-12C a first joint 63 at intermediate portion 62, and a second joint 65 at a tip portion (i.e. a portion where the tip of the foot rests) of footrest 66. Joints 63, 65 are connected via an elongate member 64, for example a rod or an elongate plate. When footrest 66 is essentially parallel to intermediate portion 62 as shown in Fig. 12B, joint 63 is closer to a heel portion of footrest 66 than to the tip portion. Joints 63, 65 may for example comprise hinges.

Through the sliding movement of base portion 60 on mounting portion 68 and the double joint between footrest 66 and intermediate portion 62, in some cases a disadvantageous positioning of a lower leg of the person may be prevented and damages to the knee may be prevented in some cases.

For example, in Fig. 12A a movement for a flexion or bending of the knee (which may be initiated by leg movement mechanism 14 of Fig. 1-5) is shown. In this case, base portion 60 as indicated by an arrow 61 slides upward (towards a head of the patient), footrest 66 moves away from intermediate portion 62 and base portion 60 as indicated by an arrow 67, and footrest 66 tilts as indicated by an arrow 72. In contrast thereto, in Fig. 12B the situation for a knee extension, i.e. a straightening of the knee, is shown. Here, base portion 60 slides downward (away from the head of the patient) on mounting portion 68 as indicated by an arrow 69, and footrest 66 is parallel and close to intermediate portion 62.

In Fig. 12C, the foot mechanism is shown in a position that corresponds to a dorsal extension of the ankle joint of a patient. Here, also base portion 60 moves slidingly on mounting portion 68 as indicated by an arrow 71, and footrest 66 tilts as indicated by an arrow 70, which corresponds to a lifting motion of the tip of the foot.

Other kinds of movements may also be enabled by the foot mechanism of Fig. 12A-12C.

It should be noted that such a foot mechanism may be a passive foot mechanism, i.e. the movement may be caused for example by the leg movement mechanism, and the feet pull/push the foot mechanism accordingly, or active components like electric motors may be provided to actively control the movements of the foot mechanism, for example movements as explained with reference to Fig. 12A-12C. In such a case, also the foot mechanism may be controlled by a control unit like control unit 50 of Fig. 11 synchronously to the leg movement mechanism.

It should be noted that joints 63, 65 of the double joint may be configured to be arrestable to fix the foot mechanism in a desired position. Furthermore, one or more resilient stoppers, for example springs, may be provided to allow for a smooth deceleration with reduced force acting on the foot when footrest 66 reaches an end of a movement range, for example the position of Fig. 12B. Such forces acting on the foot may provide an additional stimulus.

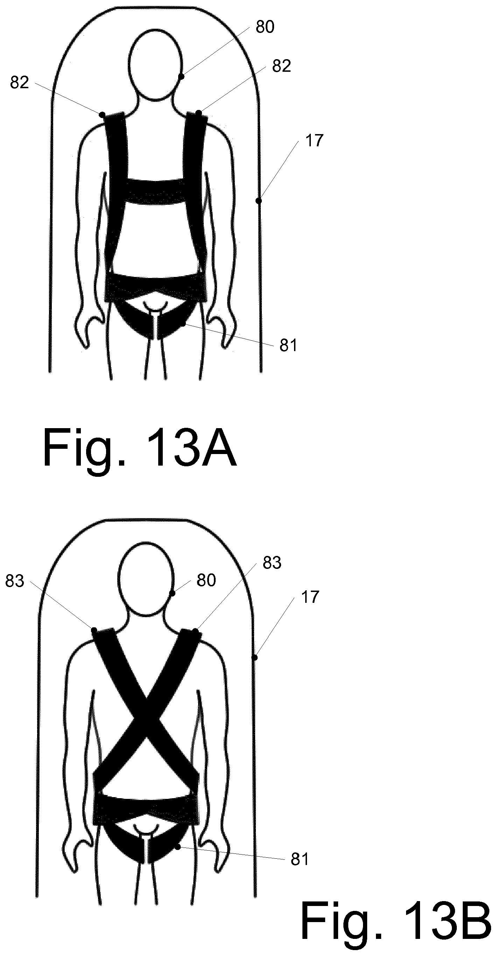

Various possibilities may be employed for fixing a patient to the apparatuses described so far with reference to Fig. 1-12. For example, a patient may be fastened to the apparatus using a harness as described in the above-mentioned EP 1 169 003 B1, where the patient is essentially suspended from above. A further possibility used in some embodiments is shown in Fig. 13A and 13B. Here, the patient is fixed to the apparatus, in particular to second table portion 17, via a pelvic harness 81. The pelvic harness 81 is directly fixed to the apparatus, for example second table portion 17. To prevent a tilting of the torso, shoulder straps may be provided. For example, in Fig. 13A straight shoulder straps 82 are provided for fixing patients 80 whereas in Fig. 13B crossed-shoulder straps 83 are provided.

In addition to this fastening mechanism which generally fastens patient 80 to the apparatus, specific body parts of the patient may be additionally fastened to the apparatus. For example, the legs may be fastened to the leg movement mechanism, the feet may be fastened to the foot mechanism, and/or a head of the patient may be additionally fastened, which may be particularly helpful for training in the vertical position. For such fastenings, for example velcro strips or similar devices may be used. Also, paddings may be used to make the patient more comfortable and to prevent pressure sores.

Also the fastenings of Fig. 13A and 13B may be used in conjunction with the previously described embodiments, but also independently therefrom.

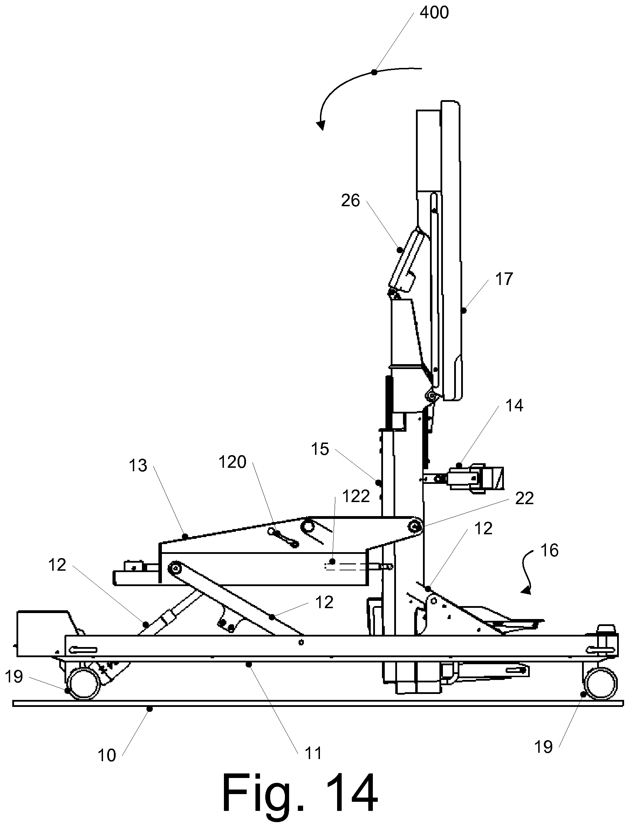

In some embodiments, an emergency lowering mechanism may be provided which may be used to lower the first and second table portions 15, 17 and therefore the patient from a vertical or tilted position to a horizontal position as shown in Fig. 1 even when actuators like drives used for tilting (or lifting) fail, for example due to a power failure. Such a mechanism is schematically shown in Fig. 14. The embodiment of Fig. 14 corresponds to the embodiment already discussed with reference to Fig. 1-5, which some additional elements as explained below. In Fig. 14, lifting mechanism 12 is only partially shown for a clearer representation of the emergency lowering mechanism.

For performing an emergency lowering of the patient from the vertical position shown in Fig. 14, a lever 120 is operated by a person, for example a therapist supervising the training. To initiate the movement from the vertical position, a gas cylinder 122 or similar, preferably power-independent actuating element is provided. When the movement has been initiated in a direction indicated by an arrow 400 and the first and second table portions 15, 17 are in a tilted position such that a centre of gravity of the patient in the view of Fig. 14 is on the left side of axis 22, the weight of the patient additionally supports and drives the movement. To prevent the movement from becoming to fast, the actuators or drives used in normal operation of the apparatus for tilting, like drive 300 of Fig. 3, may damp the movement, or additional dampers may be provided. To enable such a movement, the actuators and drives like drive 300 may be configured such that they allow a movement when e.g. power fails.

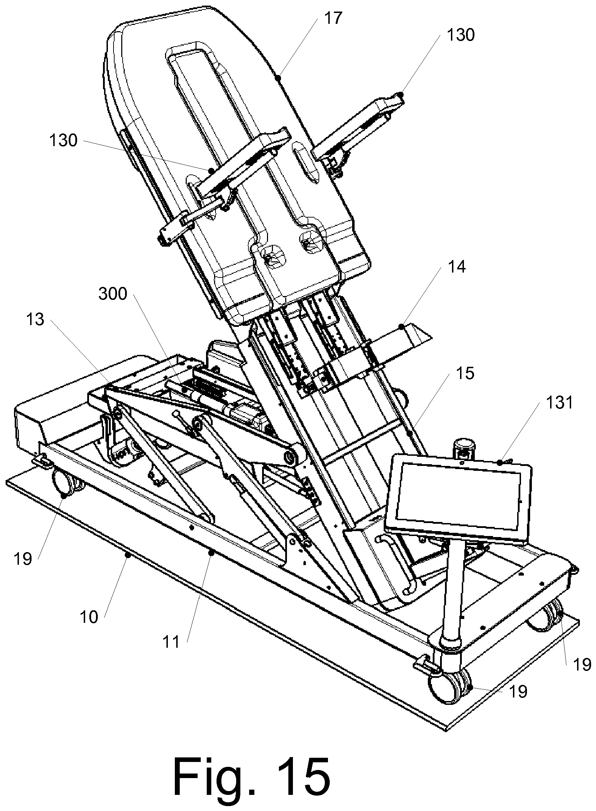

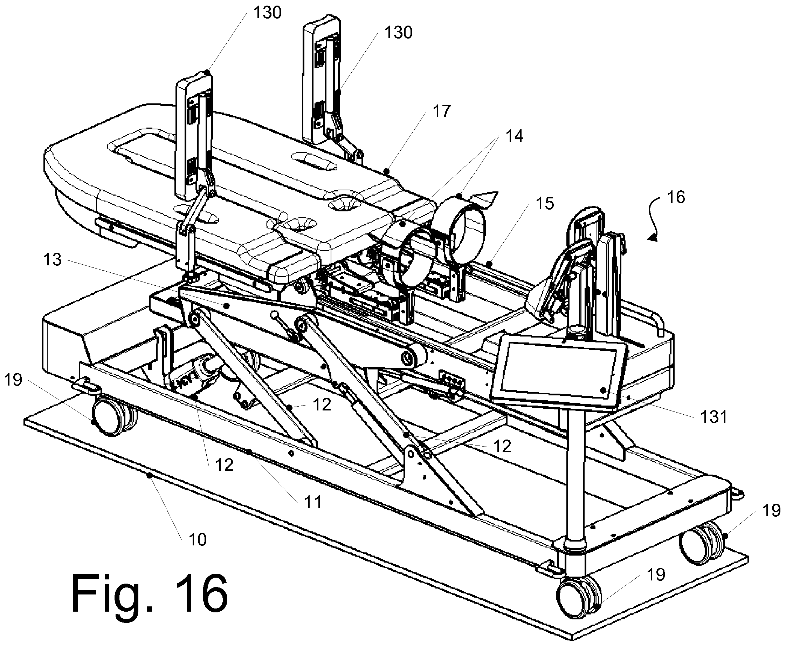

Further optional features of some embodiments are shown in Fig. 15 and 16. Fig. 15 shows a perspective view of an apparatus according to an embodiment with first table portion 15 in a position tilted about 60 degrees to horizontal, and Fig. 16 shows the embodiment in a vertical position. The embodiment of Figs. 15 and 16 is based on the embodiment of Fig. 1-5, and only additional features will be discussed in the following.

In addition to features already described, the embodiment of Fig. 15 and 16 comprisies armrests 130. Arms of a person in some embodiments may be fixed to armrests 130 using belts, Velcro straps or similar devices.

Furthermore, the embodiment of Fig. 15 and 16 comprises a panel 131 for monitoring and/or operating the apparatus. Panel 131 may comprise a so-called touchscreen to allow user input and operating of the apparatus while also being usable to display information regarding the status of the apparatus and/or of the patient training. For example, the curves of Fig. 8 may be displayed. In other embodiments, panel 131 may comprise a conventional display and other input elements like a keyboard. Panel 131 may also be located differently than shown. In still other embodiments, panel 131 may be omitted, and the apparatus may be controlled via a remote device like a computer or a handheld device which is linked to the apparatus in a wireless or wire-based manner. In still other embodiments, both a panel and a remote device may be used.

While various embodiments have been described in detail, it is emphasized again that these embodiments serve only for illustration purposes and are not to be construed as limiting the scope of the present application.

## Claims

1. An apparatus for locomotion therapy, comprising:
a base (11),
a first table portion (15),
a tilting mechanism (13,300) coupling said base (11) to said first table portion (15) and being configured to tilt said first table portion (15) between a horizontal position and a vertical position,
a foot mechanism (16) mounted to said first table portion (15) to receive feet of a patient (80), a leg movement mechanism (14; 51) configured to move the legs of the patient (80),
wherein said leg movement mechanism (14) comprises a first mechanism (14A) for a left leg of the patient (80) and a second mechanism (14B) for a right leg of the patient (80), wherein a distance between said first mechanism (14A) and said second mechanism (14B) is adjustable.

2. The apparatus of claim 1, wherein
a second table portion (17) is coupled with said first table portion (15) and adjustable with respect to said first table portion (15) to adapt the apparatus to patients (80) of different heights.

3. The apparatus of claim 2 wherein said second table portion (17) is connected to said tilting mechanism (13, 300) only via said first table portion (15).

4. The apparatus of any of claims 1-3 wherein the leg movement mechanism 14 is configured to flex knees of the patient (80).

5. The apparatus of any of claims 1-4 wherein the leg movement mechanism 14 comprise belts to fix the legs of the patient to the mechanism.

6. The apparatus of claim 5 wherein the belts may be adjustable to accommodate leg movement mechanism (14) to different leg diameters

7. The apparatus of any one of claims 1-6, further comprising a lifting mechanism (12) configured to lift said first table portion in a vertical direction.

8. The apparatus of any one of claims 1-6, wherein said foot mechanism (16) comprises, for one or each foot, a base portion (60) and a footrest (66; 35A, 35B) coupled to said base portion.

9. The apparatus of claim 8 , wherein said footrest (35) comprises a mechanic foot stimulation mechanism (37; 52),
wherein said apparatus further comprises a control unit (50) to synchronously control said leg movement mechanism (14; 51) and said foot stimulation mechanism (37; 52).

10. The apparatus of claim 8 or 9, wherein said footrest (66) is coupled with said base portion (60) via a double joint (63, 65).

11. The apparatus of claim 10, wherein said double joint comprises a first joint (63) coupled with said base, a second joint (65) coupled with said footrest (66) at a tip portion thereof, and an elongate member (64) between said first joint (63) and said second joint (65).

12. The apparatus of any one of claims 8-11, wherein said base portion (60) is slidable with respect to said first table portion (15).

13. The apparatus of any one of claims 1-12, wherein said foot mechanism (16) comprises a load sensing mechanism (36).

14. The apparatus of any one of claims 1-13, further comprising a pelvic harness (81) for receiving a pelvic region of the patient (80).

15. The apparatus of claim 14, wherein said pelvic harness (81) is coupled to said second table portion (17).

## Patentansprüche

1. Vorrichtung zur Fortbewegungstherapie, umfassend:
eine Basis (11),
einen ersten Tischabschnitt (15),
einen Neigungsmechanismus (13, 300), welcher die Basis (11) mit dem ersten Tischabschnitt (15) koppelt und welcher eingerichtet ist, den ersten Tischabschnitt (15) zwischen einer horizontalen Position und einer vertikalen Position zu neigen,
ein Fußmechanismus (16), der an dem ersten Tischabschnitt (15) angebracht ist, um Füße eines Patienten (80) zu empfangen,
einen Beinbewegungsmechanismus (14; 51), welcher eingerichtet ist, die Beine des Patienten (80) zu bewegen,
wobei der Beinbewegungsmechanismus (14) einen ersten Mechanismus (14A) für ein linkes Bein des Patienten (80) und einen zweiten Mechanismus (14B) für ein rechtes Bein des Patienten umfasst, **dadurch gekennzeichnet,**
**dass** ein Abstand zwischen dem ersten Mechanismus (14A) und dem zweiten Mechanismus (14B) einstellbar ist.

2. Vorrichtung nach Anspruch 1, wobei
ein zweiter Tischabschnitt (17) mit dem ersten Tischabschnitt (15) gekoppelt ist und bezüglich des ersten Tischabschnitts (15) einstellbar ist, um die Vorrichtung an Patienten (80) unterschiedlicher Höhe anzupassen.

3. Vorrichtung nach Anspruch 2, wobei der zweite Tischabschnitt (17) mit dem Neigungsmechanismus (13, 300) nur über den ersten Tischabschnitt (15) gekoppelt ist.

4. Vorrichtung nach einem der Ansprüche 1-3, wobei der Beinbewegungsmechanismus (14) eingerichtet ist, Knie des Patienten (80) zu beugen.

5. Vorrichtung nach einem der Ansprüche 1-4, wobei der Beinbewegungsmechanismus (14) Gurte umfasst, um die Beine des Patienten an dem Mechanismus zu fixieren.

6. Vorrichtung nach Anspruch 5, wobei die Gurte einstellbar sein können, um den Beinbewegungsmechanismus (14) an unterschiedliche Beindurchmesser anzupassen.

7. Vorrichtung nach einem der Ansprüche 1-6, weiter umfassend einen Hebemechanismus (12), welcher eingerichtet ist, den ersten Tischabschnitt in einer vertikalen Richtung zu heben.

8. Vorrichtung nach einem der Ansprüche 1-6, wobei der Fußmechanismus (16) für einen Fuß oder jeden Fuß einen Basisabschnitt (60) und eine Fußablage (66; 35A, 35B), welche mit dem Basisabschnitt gekoppelt ist, umfasst.

9. Vorrichtung nach Anspruch 8, wobei die Fußablage (35) einen mechanischen Fußstimulierungsmechanismus (37; 52) umfasst,
wobei die Vorrichtung weiter eine Steuereinheit (50) zur synchronen Steuerung des Beinbewegungsmechanismus (14; 51) und des Fußstimulierungsmechanismus (37; 52) umfasst.

10. Vorrichtung nach Anspruch 8 oder 9, wobei die Fußablage (66) über ein Doppelgelenk (63, 65) mit dem Basisabschnitt (60) gekoppelt ist.

11. Vorrichtung nach Anspruch 10, wobei das Doppelgelenk ein erstes Gelenk (63), welches mit der Basis gekoppelt ist, ein zweites Gelenk (65), welches mit der Fußablage (66) an einem spitzen Abschnitt hiervon gekoppelt ist, und ein langgestrecktes Element (64) zwischen dem ersten Gelenk (63) und dem zweiten Gelenk (65) umfasst.

12. Vorrichtung nach einem der Ansprüche 8-11, wobei der Basisabschnitt (60) gleitfähig bezüglich des ersten Tischabschnitts (15) ist.

13. Vorrichtung nach einem der Ansprüche 1-12, wobei der Fußmechanismus (16) einen Lasterfassungsmechanismus (36) umfasst.

14. Vorrichtung nach einem der Ansprüche 1-13, weiter umfassend einen Beckengurt (81) zum Empfangen eines Beckenbereichs des Patienten (80).

15. Vorrichtung nach Anspruch 14, wobei der Beckengurt (81) mit dem zweiten Tischabschnitt (17) gekoppelt ist.

## Revendications

1. Appareil de thérapie locomotrice, comprenant :
une base (11),
une première portion de table (15),
un mécanisme d'inclinaison (13, 300) couplant ladite base (11) à ladite première portion de table (15) et configuré pour incliner ladite première portion de table (15) entre une position horizontale et une position verticale,
un mécanisme pour pied (16) monté sur ladite première portion de table (15) pour recevoir les pieds d'un patient (80),
un mécanisme de mouvement de jambe (14 ; 51) configuré pour mouvoir les jambes du patient (80),
dans lequel ledit mécanisme de mouvement de jambe (14) comprend un premier mécanisme (14A) pour une jambe gauche du patient (80) et un second mécanisme (14B) pour une jambe droite du patient (80), **caractérisé en ce qu'**une distance entre ledit premier mécanisme (14A) et ledit second mécanisme (14B) est réglable.

2. Appareil selon la revendication 1, dans lequel
une seconde portion de table (17) est couplée à ladite première portion de table (15) et réglable par rapport à ladite première portion de table (15) pour adapter l'appareil à des patients (80) de différentes statures.

3. Appareil selon la revendication 2, dans lequel ladite seconde portion de table (17) est raccordée audit mécanisme d'inclinaison (13, 300) uniquement via ladite première portion de table (15).

4. Appareil selon l'une quelconque des revendications 1 à 3, dans lequel le mécanisme de mouvement de jambe (14) est configuré pour faire fléchir les genoux du patient (80).

5. Appareil selon l'une quelconque des revendications 1 à 4, dans lequel le mécanisme de mouvement de jambe (14) comprend des sangles pour fixer les jambes du patient au mécanisme.

6. Appareil selon la revendication 5, dans lequel les sangles peuvent être réglables pour ajuster le mécanisme de mouvement de jambe (14) à différents diamètres de jambe

7. Appareil selon l'une quelconque des revendications 1 à 6, comprenant en outre un mécanisme de levage (12) configuré pour lever ladite première portion de table dans une direction verticale.

8. Appareil selon l'une quelconque des revendications 1 à 6, dans lequel ledit mécanisme pour pied (16) comprend, pour un ou chaque pied, une portion de base (60) et un repose-pied (66 ; 35A, 35B) couplé à ladite portion de base.

9. Appareil selon la revendication 8, dans lequel ledit repose-pied (35) comprend un mécanisme de stimulation de pied mécanique (37 ; 52),
dans lequel ledit appareil comprend en outre une unité de commande (50) pour commander de façon synchrone ledit mécanisme de mouvement de jambe (14 ; 51) et ledit mécanisme de stimulation de pied (37 ; 52).

10. Appareil selon la revendication 8 ou 9, dans lequel ledit repose-pied (66) est couplé à ladite portion de base (60) via un double joint (63, 65).

11. Appareil selon la revendication 10, dans lequel ledit double joint comprend un premier joint (63) couplé à ladite base, un second joint (65) couplé audit repose-pied (66) au niveau d'une extrémité de bout de celui-ci, et un organe allongé (64) entre ledit premier joint (63) et ledit second joint (65).

12. Appareil selon l'une quelconque des revendications 8 à 11, dans lequel ladite portion de base (60) est coulissante par rapport à ladite première portion de table (15).

13. Appareil selon l'une quelconque des revendications 1 à 12, dans lequel ledit mécanisme de pied (16) comprend un mécanisme de détection de charge (36) .

14. Appareil selon l'une quelconque des revendications 1 à 13, comprenant en outre un harnais pelvien (81) pour recevoir une région pelvienne du patient (80).

15. Appareil selon la revendication 14, dans lequel ledit harnais pelvien (81) est couplé à ladite seconde portion de table (17).
